## Europäisches Patentamt
## European Patent Office
## Office européen des brevets

(19)

(11) Publication number: **0 008 498**
**A1**

(12)

# EUROPEAN PATENT APPLICATION

(21) Application number: **79301464.8**

(22) Date of filing: **24.07.79**

(51) Int. Cl.³: **C 12 N 11/08**
**C 12 P 19/02, C 12 P 19/24**

(30) Priority: **15.08.78 GB 3337478**

(43) Date of publication of application:
**05.03.80 Bulletin 80/5**

(84) Designated Contracting States:
**AT BE CH DE FR GB IT LU NL SE**

(71) Applicant: **IMPERIAL CHEMICAL INDUSTRIES LIMITED**
**Imperial Chemical House Millbank**
**London SW1P 3JF(GB)**

(72) Inventor: **Corcoran, Eugene Gerard**
**47 Ridley Drive Norton**
**Stockton-on-Tees, Cleveland(GB)**

(72) Inventor: **Bentley, John**
**38 Conway Road Taplow**
**Maidenhead, Berkshire(GB)**

(72) Inventor: **Thompson, Morice William**
**Urishay Highfield Lane**
**Cocks Green Maidenhead, Berkshire(GB)**

(74) Representative: **Aufflick, James Neil**
**Imperial Chemical Industries Limited Legal Department:**
**Patents Thames House North Millbank**
**London SW1P 4QG(GB)**

(54) Immobilized enzyme preparation and process for its production.

(57) An immobilized enzyme preparation, its production and its use in which an enzyme is immobilized on particles having amino groups on their surfaces, a dispersion of the particles being produced by a specified process. Preferably the particles comprise inert inorganic fillers such as sand encapsulated in polyamide polymers or copolymers. The enzyme preparation may be used in the following enzyme catalysed conversions:-

lactose to glucose and galactose; glucose to fructose; and starch to glucose.

EP 0 008 498 A1

TITLE MODIFIED.
see front page.

Immobilized Enzyme Preparation

This invention relates to an immobilised enzyme preparation, a process for the production of such a preparation and the use of such a preparation in an enzyme catalysed reaction.

Enzymes are proteins which catalyse chemical reactions. However their utility for various applications has been limited since it is difficult to separate enzymes from reaction media, thus limiting product purity. Moreover soluble enzymes may generally be used only once in a batch reaction and, because of the high costs of many enzymes, their industrial use has been limited, even though they are extremely efficient catalysts. In the past decade much research effort has been devoted to devising techniques for immobilizing enzymes by attaching or bonding them to water-insoluble carrier materials in such a way that the enzymes are rendered immobile yet remain catalytically active. Known immobilised enzyme preparations have several disadvantages, for example some when packed into a column exhibit unsatisfactory flow properties or low enzyme yields or decreased stability. Thus an increasing need exists to develop improved immobilized enzyme preparations.

Our co-pending UK Application No. 32398/77 (corresponding to Belgian Pat. 869317) describes an improved immobilized enzyme preparation comprising a support material, the surface of which carries hydrophobic groups, and having molecules of at least one enzyme attached to the hydrophobic groups. Whilst, in this enzyme preparation, the support material may be in any suitable physical form, we have found that particulate materials prepared in a specific manner give improved results.

In our UK Specifications Nos. 1,373,531; 1,403,794; 1,419,199; 1,453,713; 1,506,221; 1,506,222; and our UK Application No. 19487/76 (which corresponds to US Serial No. 795307 and German OLS 2721501) we describe a number of variants of a process (hereinafter referred to as the dispersion preparation process) for the preparation of a stable dispersion in a hydrocarbon liquid of, inter alia, a polyamide which is insoluble in that liquid, by thermal polymerisation in the hydrocarbon liquid of one or more polyamide-forming reactants, at least one of said reactants being insoluble in the hydrocarbon liquid at the polymerisation temperature, the process comprising the steps of:

(1)      bringing said insoluble reactant or reactants into the state of a stable dispersion of finely divided particles in the hydrocarbon liquid with the aid of a dispersing agent which is soluble in the hydrocarbon and which incorporates in the molecule (a) a polymeric component which is solvatable by the hydrocarbon and (b) a grouping which is capable of becoming associated with the particles of the insoluble reactant(s),

(2)      heating the dispersion of the insoluble reactant(s), together with any other necessary reactants which are soluble in the hydrocarbon liquid, to the polymerisation temperature in the presence of a polymer-stabilizing agent which is soluble in the hydrocarbon and incorporates in the molecule (i) a polymeric component which is solvatable by the hydrocarbon and (ii) a grouping which is capable of becoming associated with the particles of the resulting polyamide as that polymer is formed.

The dispersion preparation process is also described in "Dispersion polymerisation in organic media", Editor K E J Barrett, Published by J Wiley.

According to the present invention we provide an immobilized enzyme preparation in which molecules of at least one enzyme are attached to amino-groups on the surface of a particulate support material wherein the support material comprises particles which have amino-groups on their surfaces and which have been separated from a dispersion produced by a dispersion preparation process (as hereinbefore defined).

Further according to the invention we provide a process for the production of an immobilized enzyme preparation which comprises the steps of:

(1)     producing a stable dispersion of particles having aminogroups on their surfaces by a dispersion preparation process (as hereinbefore defined).

(2)     separating the particles from the dispersion, and

(3)     contacting the particles with at least one enzyme for such a time and under such conditions that enzyme molecules become attached to amino-groups on the surfaces of the particles.

Further according to the invention we provide a process for performing an enzyme-catalysed reaction wherein the enzyme is in the form of an immobilized enzyme preparation in which molecules of at least one enzyme are attached to amino-groups on the surface of a particulate support material, the support material comprising particles which have amino-groups on their surfaces and which have been separated from a dispersion produced by a dispersion preparation process (as hereinbefore defined).

Variants of the dispersion preparation process which can be used to prepare the dispersion include the following:-

(a)     A variant wherein the insoluble reactant is liquifiable at temperatures below the polymerisation temperature, in which case step (1) of the dispersion preparation process produces an emulsion of liquid reactant particles in the hydrocarbon (UK Specification No. 1,373,531).

(b)     A variant wherein the insoluble reactant is solid at the polymerisation temperature, in which case step (1) of the dispersion preparation process produces a dispersion of solid reactant particles in the hydrocarbon (UK Specification No. 1,403,794).

(c)     A variant wherein the insoluble reactant, which can be either as in (a) or as in (b) above, is soluble in a second, inert, liquid which is immiscible with the hydrocarbon and can be separated therefrom by distillation, the resulting solution then being emulsified in the hydrocarbon-analogous to (a) above (UK Specification No. 1,419,199).

(d)      A variant wherein a single dispersing and/or polymer-stabilizing agent is used in both steps (1) and (2) of the dispersion preparation process (above patent references).

(e)      A variant wherein two insoluble solid reactants are treated together as in (b) above to give a dispersion of a co-polymer (UK Specification No. 1,506, 221).

(f)      A variant wherein a solid, inert modifier is co-dispersed with the insoluble, solid reactant(s) in step (1) of the dispersion preparation process performed as in (b) or (e) above, to give composite disperse particles (UK Specification No. 1,506,222).

(g)      A variant wherein a solid inert modifier is introduced into the emulsified insoluble reactant(s) in step 1 of the dispersion preparation process performed as in (a) or (c) above, to give composite disperse particles (UK Specification No. 1,453,713).

(h)      In a modification of either variant (a) or variant (c), instead of using a pre-formed dispersant/stabiliser, this may be formed _in situ_ during the polymerisation from a suitable polymeric precursor (supplying the solvatable component) on to which some of the polymerizing reactant(s) become grafted (UK Appln. No. 19487/76).

Preferably the polyamide-forming reactants are 6-amino-caproic acid, nylon 66 salt or 11-amino undecanoic acid, the preferred polyamides being homopolymers or co-polymers of these with other materials, in particular diethylene triamine. A particularly suitable polymer is one formed from nylon 66 salt and diethylene triamine. The particles used in the support material may be formed entirely from polyamide polymers and co-polymers but are preferably modified particles comprising an inert inorganic filler encapsulated in a polyamide polymer or co-polymer. Suitable fillers include sand and particles of glass, magnetite and titanium dioxide and impart to the particles of the immobilized enzyme preparation a suitable density. The filled particles are produced in dispersion by the dispersion preparation process as described in (f) or (g) above.

Dispersing agents and polymer-stabilising agents used in the dispersion preparation process may be any of those which satisfy the requirements set out in steps (1) and (2) of the process respectively as defined above. It is preferred to use a single agent which can perform both functions, and a particularly suitable such agent is a graft co-polymer one polymer component of which is derived from poly (12-hydroxystearic acid) and is per se soluble in the hydrocarbon liquid, and another polymeric component of which is an acrylic polymer chain which is capable of becoming associated with the particles of the insoluble reactant(s) and in turn with the resulting polyamide as it is formed. The hydrocarbon used is preferably an aliphatic hydrocarbon boiling within the range 150 - 300°C, preferably 180 - 250°C.

In step (1) of the dispersion preparation process, the polyamide-forming reactants are brought into an emulsified liquid state or are dispersed by a grinding or milling procedure (such as ball milling), in the hydrocarbon liquid in the presence of the dispersing agent, preferably until the mean particle size of reactant is within the range 5 - 200 μ, especially 10 - 50 μ. Very suitably in step (1) an additional polyfunctional amino-compound is included with the reactants. This gives added amino groups in the final polymer and also facilitates the performance of step (2). The additional amino-compound preferably has at least three groups reactive in the polyamide-forming process, tris (aminomethyl) propane and, especially, diethylene triamine being examples of suitable compounds for this purpose.

In step (2) of the dispersion preparation process, the dispersion of insoluble reactants is heated to the polymerisation temperature in the particular case. Generally speaking this temperature will be between 150 and 300°C. If a filler is present a composite particle comprising the filler with a polymer or co-polymer coating will result.

By suitable operation of the dispersion preparation process a stable dispersion is formed of particles of regular shape and having a size and density suitable for the particular use to which the immobilized enzyme preparation is to be put, i.e. particular enzyme reaction and mode of operation thereof - e.g. fluidized bed, column etc. The particles are then separated from the dispersion liquid e.g. by filtration or centrifuging, and are then washed with any suitable solvent, e.g. a xylene/toluene mixture in order to remove high boiling hydrocarbon, which is preferably followed by further treatment, e.g. washing with an alcohol such as methanol or ethanol, so as to facilitate working in an aqueous medium, before being contacted with the enzyme.

To enable an enzyme to be attached to the particulate support material, the material is treated with a compound which (a) modifies amino-groups on its surface or (b) with a bifunctional compound which is capable of reacting with said amino-groups and also with the enzyme. With regard to (b), examples of suitable bifunctional compounds include glutaraldehyde and isocyanates. Regarding (a), the amino groups may be converted into, e.g. their bromoacetal or diazonium derivatives, using suitable reagents, the material having these modified functional groups is then contacted with an enzyme.

To a suspension of treated particulate support material in an aqueous medium can be added an aqueous solution of the enzyme to be immobilized. Molecules of the enzyme attach themselves to support particles to give the immobilized enzyme preparation which is then - separated from the suspension, e.g. by filtration.

The immobilized enzyme preparation may be used in any suitable manner, for example in fluidised beds or in columns, when acting as the catalyst in the enzyme-catalysed reaction. Enzymes which can usefully be immobilized include lactase, glucose isomerase and amylo-glucosidase (glucoamylase). Enzyme catalysed reactions include the following conversions:-

Lactose to glucose and galactose

Glucose to fructose

Starch to glucose

and other enzymic reactions referred in our UK Specification No. 1,497,888. The conversion of glucose to fructose can be carried out as described in our UK Specifications Nos. 1,451,273, 1,492,258 and 1,497,888. The conversion of lactose to glucose and galactose can be carried out as described in our UK Specification No. 1,531,303.

The invention is illustrated by the following Examples:-

EXAMPLE 1

The following ingredients were charged to a 2 gallon laboratory ball mill together with the requisite quantity of steatite balls.

| | |
|---|---|
| Aliphatic hydrocarbon (Exxon Trade Mark Isopar M) | 2 L |
| 6 - Amino Caproic Acid | 350 g |
| Diethylene triamine | 12 g |
| Graft co-polymer dispersant solution | 50 g |

The charge was ground for 10 hours and transferred to a laboratory reactor fitted with a stirrer, thermometer and Dean and Stark separator. The contents of the reactor were heated to reflux temperature and 48 ml of water was removed over a period of 30 minutes. While the water was being collected, 300 ml of a 3% by weight dispersant solution in the aliphatic hydrocarbon described above was added at a rate of 10 ml/min to the contents of the reactor. This dispersant feed during the reaction assisted particle size control. The resultant product of the reaction, was filtered, washed in a 50/50 mixture of zylene/toluene, then filtered again and finally washed in hot ethanol. The mean partical size was 25 $\mu$.

The graft co-polymer dispersant solution used in this example was a 37% by weight solution in the aliphatic hydrocarbon described above of a poly-12-hydroxy stearic acid/poly-ethyl acrylate/methacrylic acid co-polymer (50/45/5 w/w/w). This was obtained by reacting poly (12-hydroxy stearic acid) of acid value 33 mg KOH/g with glycidyl methacrylate until the product had substantially zero acid value; then co-polymerising the resulting ester at a ratio 1:1 by weight with a mixture of ethyl acrylate and methacrylic acid in the weight proportions 90:10.

EXAMPLE 2

The following ingredients were charged to a 2 gallon ball mill.

| | |
|---|---|
| Aliphatic hydrocarbon (Exxon Trade Mark Isopar L) | 2 L |

| | |
|---|---|
| Nylon 6.6 Salt | 350 g |
| Diethylene triamine | 12 g |
| Graft co-polymer dispersant solution | 50 g |
| (as described in example 1) | |

The grinding and product preparation were as in example 1.

EXAMPLE 3

The following ingredients were charged to a 2 gallon ball mill.

| | |
|---|---|
| Aliphatic hydrocarbon (Exxon Trade Mark Isopar M) | 2 L |
| β-Cristobalite (a preferred type of sand) | 726 g |
| Amino caproic acid | 350 g |
| Diethylene triamine | 12 g |
| Graft Co-polymer dispersant | 50 g |
| (as described in example 1) | |

The charge was ground for 20 hours and the product prepared as in example 1. The mean particle size was about 20 $\mu$.

EXAMPLE 4

The following ingredients were charged to a 2 gallon ball mill.

| | |
|---|---|
| Aliphatic hydrocarbon (Exxon Trade Mark Isopar L) | 2 L |
| β-Cristobalite | 726 g |
| Nylon 6.6 Salt | 350 g |
| Diethylene triamine | 12 g |
| Graft co-polymer dispersant | 50 g |
| (as described in example 1) | |

The charge was ground and the product prepared as in example 3.

EXAMPLE 5

The following ingredients were charged to a 2 gallon ball mill.

| | |
|---|---|
| Aliphatic hydrocarbon (Exxon Trade Mark Isopar L) | 2 L |
| β-Cristobalite | 726 g |
| Nylon 6.6 Salt | 200 g |
| Diethylene triamine | 12 g |
| Graft co-polymer dispersant | 25 g |

(as described in example 1) The charge was ground and the product prepared as in example 3.

EXAMPLE 6

The following ingredients were charged to a 2 gallon ball mill.

| | |
|---|---|
| Aliphatic hydrocarbon (Exxon Trade Mark Isopar L) | 2 L |
| β-Cristobalite | 726 g |
| Nylon 6.6 Salt | 180 g |
| Amino caproic acid | 20 g |
| Diethylene triamine | 12 g |
| Graft co-polymer dispersant | 25 g |

The charge was ground and the polymer prepared as in example 3.

EXAMPLE 7

The following ingredients were charged to a 2 gallon laboratory ball mill together with the requisite quantity of steatite balls.

| | |
|---|---|
| Aliphatic hydrocarbon (Exxon Trade Mark Isopar L) | 1.5 L |
| Nylon 6.6 salt | 280 g |
| β-Cristobalite | 1000 g |
| Graft co-polymer dispersant solution | 14 g |

The charge was ground for 8 hours and, together with an extra 1.5 L of Isopar L and 50 mls of hexamethylene diamine, was transferred to a laboratory reactor fitted with a stirer, thermometer and Dean and Stark separator. The contents of the reactor were heated to reflux temperature and 44 ml of water was removed over a period of 30 minutes. The resultant product of the reaction, was filtered, washed in a 50/50 mixture of xylene/toluene, then filtered again and finally washed in hot ethanol. The mean partical size was 25 μ.

The graft co-polymer dispersant solution used in this example was a 37% by weight solution in the aliphatic hydrocarbon described above of a poly-12-hydroxy stearic acid/poly-ethyl acrylate/methacrylic acid co-polymer (50/45/5 w/w/w). This was obtained by reacting poly (12-hydroxy stearic acid) of acid value 33 mg KOH/g with glycidyl methacrylate until the product had substantially zero acid value; then co-polymerising the resulting ester at a ratio 1:1 by weight with a mixture of ethyl acrylate and methacrylic acid in the weight proportions 90:10.

EXAMPLE 8

The particles prepared in examples 1 - 7 had similar enzyme binding capacities, however for reasons of convenience particles which contain fillers such as β-Crystobalite, as prepared in examples 4 - 6 are preferred.

2 g of particles prepared as in each of examples 1 - 6 were treated with 10 ml of a 5% glutaraldehyde solution for ½ hour at pH 7.0. In each case the resultant aldehyde derivative was then filtered, washed with 10 ml of water and reacted with 10 ml of a β-Galactosidase enzyme solution* extracted from β-Coagulans NRRL B-8100 (obtained from Reynolds Tobacco Co.). The immobilized enzyme was then packed into a column. A 5% lactose solution in 50 mM phosphate buffer at pH 6.5 was passed at $50^{\circ}$C through the column. After 36 hours, 100 grms. of lactose was hydrolysed.

EXAMPLE 9

50 g of particles prepared as in each of examples 1 - 7 were treated with 250 ml of a 5% glutaraldehyde solution for ½ hour at pH 2.0. In each case resultant aldehyde derivative was then filtered, washed with 250 ml of water and reacted with 250 mls of a β-galactosidase enzyme solution* as in Example 8. The particles were then packed into a fluidised bed reactor and a 5% lactose solution made up in 50 mM phosphate buffer at pH 6.5 was passed through a reactor at a rate sufficient to fluidise the immobilised enzyme particles. After 100 hours, 300 grms. of lactose had been hydrolysed.

EXAMPLE 10

10 ml of a solution* of an amyloglucosidase enzyme purchased from Glaxo was immobilized and treated with a 5% starch solution (20% DE) at pH 4.5 as in example 9. After 100 hours 2000g of starch was hydrolysed.

EXAMPLE 11

250 ml of a solution* of an amyloglucosidase enzyme purchased from Glaxo was immobilized and treated with a 5% starch solution (20% DE) at pH 4.5 as in example 9. After 100 hours 2000g of starch was hydrolysed.

*Note    In the enzyme solutions used in Examples 8 - 11 total protein concentration was 50 mgs/ml.

PA/JNA/MP
13 July 1979

1.      An immobilized enzyme preparation in which molecules of at least one enzyme are attached to amino-groups on the surface of a particulate support material wherein the support material comprises particles which have amino-groups on their surfaces and which have been separated from a dispersion produced by a dispersion preparation process for the preparation of a stable dispersion in a hydrocarbon liquid by thermal polymerisation in the hydrocarbon liquid of one or more polyamide-forming reactants, at least one of said reactants being insoluble in the hydrocarbon liquid at the polymerisation temperature, the dispersion preparation process comprising the steps of:

(A)      bringing said insoluble reactant or reactants into the state of a stable dispersion of finely divided particles in the hydrocarbon liquid with the aid of a dispersing agent which is soluble in the hydrocarbon and which incorporates in the molecule (a) a polymeric component which is solvatable by the hydrocarbon and (b) a grouping which is capable of becoming associated with the particles of the insoluble reactant(s), and

(B)      heating the dispersion of the insoluble reactant(s) together with any other necessary reactants which are soluble in the hydrocarbon liquid, to the polymerisation temperature in the presence of a polymer-stabilizing agent which is soluble in the hydrocarbon and incorporates in the molecule (i) a polymeric component which is solvatable by the hydrocarbon and (ii) a grouping which is capable of becoming associated with the particles of the resulting polyamide as that polymer is formed.

2.      An immobilized enzyme preparation according to claim 1 wherein the support particles comprise an inert inorganic filler encapsulated in a polyamide polymer or co-polymer.

3.      An immobilized enzyme preparation according to claim 2 wherein the filler is sand, glass, magnetite or tetanium dioxide.

4.      An immobilized enzyme preparation according to any one of claims 1 to 3 wherein the enzyme is lactase, glucose isomerase or amyloglucosidase.

5.      An immobilized enzyme preparation according to any one of claims 1 to 4 wherein the polyamide is a homopolymer or co-polymer
of 6-amino-caproic acid, nylon 6.6 salt or 11-amino undecanoic acid
with diethylene triamine.

6.      A process for the production of an immobilized enzyme
preparation which comprises the steps of:

(1)     producing a stable dispersion of particles having amino-
groups on their surfaces by a dispersion preparation process for
the preparation of a stable dispersion in a hydrocarbon liquid by
thermal polymerisation in the hydrocarbon liquid of one or more
polyamide-forming reactants, at least one of said reactants being
insoluble in the hydrocarbon liquid at the polymerisation temperature, the dispersion preparation process comprising the steps of:

(A)     bringing said insoluble reactant or reactants into the
state of a stable dispersion of finely divided particles in the
hydrocarbon liquid with the aid of a dispersing agent which is soluble in the hydrocarbon and which incorporates in the molecule (a)
a polymeric component which is solvatable by the hydrocarbon and (b)
a grouping which is capable of becoming associated with the particles of the insoluble reactants(s), and

(B)     heating the dispersion of the insoluble reactant(s), together with any other necessary reactants which are soluble in the
hydrocarbon liquid, to the polymerisation temperature in the presence of a polymer-stabilizing agent which is soluble in the hydrocarbon and incorporates in the molecule (i) a polymeric component
which is solvatable by the hydrocarbon and (ii) a grouping which is
capable of becoming associated with the particles of the resulting
polyamide as that polymer is formed,

(2)     separating the particles from the dispersion, and

(3)     contacting the particles with at least one enzyme for such
a time and under such conditions that enzyme molecules become
attached to amino-groups on the surfaces of the particules.

7.      A process according to claim 5 wherein the polyamide forming reactant is 6-amino-caproic acid, nylon 6.6 salt or 11-amino
undecanoic acid.

8.      A process according to claim 6 wherein there is used as a dispersing agent and as a polymer-stabilizing agent a graft co-polymer, one polymeric component of which is derived from poly 1,2-hydroxystearic acid and is soluble in the hydrocarbon liquid, and another polymeric component of which is an acrylic polymer chain which is capable of becoming associated with the particles of the insoluble reactant(s) and in turn with the resulting polyamide as it is formed.

9.      A process according to any one of claims 6 to 8 wherein the hydrocarbon is an aliphatic hydrocarbon boiling within the range 150 - 300°C.

10.      A process for performing an enzyme-catalysed reaction wherein the enzyme is in the form of an immobilized enzyme preparation in which molecules of at least one enzyme are attached to amino-groups on the surface of a particulate support material, the support material comprising particles which have amino-groups on their surfaces and which have been separated from a dispersion produced by a dispersion preparation process for the preparation of a stable dispersion in a hydrocarbon liquid by thermal polymerisation in the hydrocarbon liquid of one or more polyamide-forming reactants, at least one of said reactants being insoluble in the hydrocarbon liquid at the polymerisation temperature, the dispersion preparation process comprising the steps of:

(A)      bringing said insoluble reactant or reactants into the state of a stable dispersion of finely divided particles in the hydrocarbon liquid with the aid of a dispersing agent which is soluble in the hydrocarbon and which incorporates in the molecule (a) a polymeric component which is solvatable by the hydrocarbon and (b) a grouping which is capable of becoming associated with the particles of the insoluble reactant(s), and

(B)      heating the dispersion of the insoluble reactant(s), together with any other necessary reactants which are soluble in the hydrocarbon liquid, to the polymerisation temperature in the presence of a polymer-stabilizing agent which is soluble in the hydrocarbon and incorporates in the molecule (i) a polymeric component which is solvatable by the hydrocarbon and (ii) a grouping which is capable of becoming associated with the particles of the resulting polyamide as that polymer is formed.

11.      A process according to claim 10 in which glucose is converted to fructose, lactose is converted into glucose and galactose or starch is converted into glucose.

0008498

**European Patent Office**

**EUROPEAN SEARCH REPORT**

Application number

EP 79 30 1464

| | **DOCUMENTS CONSIDERED TO BE RELEVANT** | | | **CLASSIFICATION OF THE APPLICATION (Int. Cl. ³)** |
|---|---|---|---|---|
| Category | Citation of document with indication, where appropriate, of relevant passages | | Relevant to claim | |
| D | GB - A - 1 506 222 (IMPERIAL CHE-MICAL INDUSTRIES)<br><br> * Claims; page 4, lines 1-3,27-33 and 112-118; page 6, lines 75-91; page 7, lines 74-78; page 8, lines 80-91; page 9, lines 89-103 *<br><br>-- | | 1-11 | C 12 N 11/08<br>C 12 P 19/02<br>C 12 P 19/24 |
| | DE - A - 2 450 132 (IMPERIAL CHE-MICAL INDUSTRIES)<br><br> * Claims; page 2, line 22; example 1 *<br><br>---- | | 1-11 | |

**TECHNICAL FIELDS SEARCHED (Int.Cl. ³)**

C 12 N 11/00

**CATEGORY OF CITED DOCUMENTS**

X: particularly relevant

A: technological background

O: non-written disclosure

P: intermediate document

T: theory or principle underlying the invention

E: conflicting application

D: document cited in the application

L: citation for other reasons

&: member of the same patent family, corresponding document

The present search report has been drawn up for all claims

| Place of search | Date of completion of the search | Examiner |
|---|---|---|
| The Hague | 30-11-1979 | RYCKEBOSCH |

EPO Form 1503.1 06.78